# EUROPEAN PATENT SPECIFICATION

(11) **EP 1 925 615 B1**
(45) Date of publication and mention of the grant of the patent: **15.07.2015**
(21) Application number: 06291762.0
(22) Date of filing: 14.11.2006
(51) Int. Cl.: C07D 451/02

(54) **Method of fluorine-18 labelling of tropane derivatives**
Verfahren zur Fluor-18 Markierung von Tropan Derivaten
Methode de marquage de fluor-18 des derives du tropane

(43) Date of publication of application: 28.05.2008
(73) Proprietor: Orphachem, 63000 Clermont-Ferrand (FR); Commissariat à l'Énergie Atomique et aux Énergies Alternatives, 75015 Paris (FR); L'UNIVERSITE DE TOURS, 37041 Tours Cedex 1 (FR)
(72) Inventor: Dolle, Frédéric, 91940 Gometz-le-Chatel (FR); Emond, Patrick, 37210 Vouvray (FR); Le Gailliard, Joël, 63160 Reignat (FR); Hinnen, Françoise, 91430 Vauhallan (FR); Helfenbein, Julie, 63000 Clermond-Ferrand (FR)
(74) Representative: Colombet, Alain André

(56) References cited:
- WO-A-96/39198
- US-A- 5 698 179
- US-B1- 6 344 179

## Description

The invention relates to a one-step [¹⁸F]radiolabelling process of tropanamine derivatives which are useful for imaging the neuronal dopamine transporter (DAT) with Positron Emission Tomography (PET).

Several affections, including Parkinson's disease, schizophrenia, attention deficit disorder or drug abuse, are related to abnormalities within the brain's dopamine system. In particular, the neuronal dopamine transporter, a membrane-bound presynaptically located protein, plays a key role in regulating the synaptic concentration of dopamine at nerve terminals and thus dopamine neurotransmission in the brain. Taking into account that DAT density can be considered as a marker of the integrity and number of presynaptic striatal dopamine-producing neurons, considerable efforts have been spent in recent years on the design and development of DAT-selective radioligands as *in vivo* Positron Emission Tomography (PET) imaging tools for the study of these illnesses and disorders as well as the evaluation of the degree of success of their treatment.

Certain tropane-type derivatives including cocaine represent today the largest class of molecules ever developed for imaging the DAT with PET and therefore labelled with a positron-emitting radioisotope.

From a radiochemical point of view, many of these derivatives have been labelled with the short-lived radioisotope carbon-11 (half-life: 20.38 min) at their *N*-methyl group or methyl ester function using [¹¹C]methyl iodide or [¹¹C]methyl triflate (for example, β-CIT or β-CCT). Also, a relatively large number of these derivatives have been labelled with fluorine-18 (half-life: 109.8 min), today the most widely used positron-emitting radioisotope, by aliphatic nucleophilic fluorination (for example, FE-CNT or β-FE-CCT) or aromatic electrophilic fluorination (β-CFT). Finally, a few of these derivatives have been labelled with bromine-76, a longer-lived positron-emitter (half-life: 16.1 h), by electrophilic bromination (for example, CBT) (Table 1).

**Table 1**

| | | | | | |
|---|---|---|---|---|---|
| 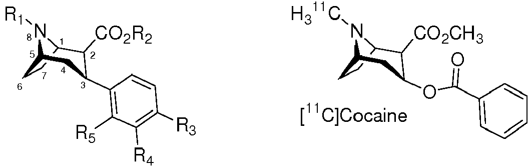 | | | | | |

| **Code** | **R₁** | **R₂** | **R₃** | **R₄** | **R₅** |
|---|---|---|---|---|---|
| β-CFT (WIN 35,428) | [¹¹C]CH₃ | [¹¹C]CH₃ | [¹⁸F]^{F} | H | H |
| β-CIT (RTI-55) | [¹¹C]CH₃ | [¹¹C]CH₃ | I | H | H |
| β-CCT (RTI-31) | CH₃ | [¹¹C]CH₃ | Cl | H | H |
| β-CMT (RTI-32) | CH₃ | [¹¹C]CH₃ | CH₃ | H | H |
| FECNT | CH₂CH₂[¹⁸F]F | CH₃ | Cl | H | H |
| β-CCT-FP (β-FCT) | (CH₂)₂CH₂[¹⁸F]F | CH₃ | Cl | H | H |
| β-CBT | CH₃ | CH₃ | [⁷⁶Br]Br | H | H |
| β-FECT (β-FE-CCT) | CH₃ | CH₂CH₂[¹⁸F]F | Cl | H | H |
| β-FETT (β-FE-CMT) | CH₃ | CH₂CH₂[¹⁸F]F | CH₃ | H | H |
| β-FIPCT (β-FiP-CCT) | CH₃ | CH(CH₃)CH₂[¹⁸F]F | Cl | H | H |
| β-CFT-FE | CH₂CH₂[¹⁸F]F | CH₃ | F | H | H |
| β-CFT-FP | (CH₂)₂CH₂[¹⁸F]F | CH₃ | F | H | H |
| β-CBT-FE | CH₂CH₂F | CH₃ | [⁷⁶Br]Br | H | H |
| β-CIT-FP (FP-CIT) | (CH₂)₂CH₂[¹⁸F]F | [¹¹C]CH₃ | I | H | H |
| β-CMT-FP (FP-CMT) | (CH₂)₂CH₂[¹⁸F]F | CH₃ | CH₃ | H | H |
| β-CBT-FP (FP-CBT) | (CH₂)₂CH₂[¹⁸F]F | CH₃ | [⁷⁶Br]Br | H | H |
| β-CDCT | CH₃ | [¹¹C]CH₃ | Cl | Cl | H |
| β-IP-CIT (RTI-121) | [¹¹C]CH₃ | CH(CH₃)₂ | I | H | H |
| NS-2214 (BMS-204756) | [¹¹C]CH₃ | *see below^{(a)}* | Cl | Cl | H |
| β-CIT-FE | CH₂CH₂F | [¹¹C]CH₃ | I | H | H |
| β-CpFMT | CH₃ | CH₃ | CH₂[¹⁸F]F | H | H |
| β-CmFMT | CH₃ | CH₃ | H | CH₂[¹⁸F]F | H |
| β-CoFMT (o-FWIN) | CH₃ | CH₃ | H | H | CH₂[¹⁸F]F |
| β-CPPIT | [¹¹C]CH₃ | *see below^{(b)}* | Cl | H | H |
| FCT | 4-[¹⁸F]FBn | CH₂CH₃ | Cl | H | H |
| E-IACFT (Altropane) | CH₂CH=CHI | [¹¹C]CH₃ | F | H | H |
| MCL301 | CH₃ | CH₂CH₂[¹⁸F]F | I | H | H |
| MCL322 | CH₃ | CH₂CH₂[¹⁸F]F | Br | H | H |
| FE@CIT | CH₃ | CH₂CH₂[¹⁸F]F | I | H | H |
| FBCINT | CH₂(CH=CH)CH₂[¹⁸F]F | CH₃ | Cl | H | H |
| FE-3FPT | CH₂CH₂[¹⁸F]F | CH₃ | 3'-furyl | H | H |
| PE2I | CH₂CH=CHI | [¹¹C]CH₃ | CH₃ | H | H |
| 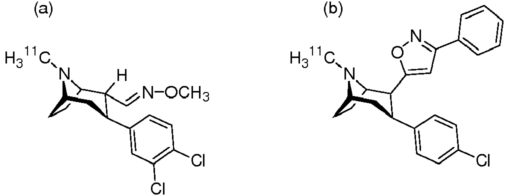 | | | | | |

Today, fluorine-18, beyond its adequate physical and nuclear characteristics, appears as the most attractive positron-emitting radioisotope for radiopharmaceutical chemistry and PET imaging, part of this continuous growing interest probably due to the successful use in clinical oncology of [¹⁸F]FDG, actually the most widely used PET-radiopharmaceutical.

Briefly, fluorine-18 displays simple decay- and emission properties with a high 97% positron abundance. Compared with the other conventional short-lived positron-emitting radionuclides carbon-11, nitrogen-13 and oxygen-15, fluorine-18 has a relatively low positron energy (maximum 635 keV) and the shortest positron linear range in tissue (2.3 mm), resulting in the highest resolution in PET imaging. Its half-life (109.8 min) is long enough to give access to relatively extended imaging protocols compared to what is possible with carbon-11, therefore facilitating kinetic studies and high-quality plasma metabolite analysis. Moreover, from a chemical point of view, fluorine-18 allows for multi-step synthetic approaches that can be extended over hours. Finally, fluorine-18 can be reliably and routinely produced at the multi-Curie level, using the well-characterized (p,n) nuclear reaction on an oxygen-18-enriched water target on widely implemented biomedical cyclotrons of a relatively low-energy proton beam (e.g., 18 MeV). This distinctive advantage, combined with its favourable half-life, permits the transport and the use of fluorine-18-labelled radiopharmaceuticals (such as the archetype [¹⁸F]FDG) at 'satellite' PET units without an on-site cyclotron production facility.

LBT-999 (1,8-((E)-4-fluoro-but-2-enyl)-3β-p-tolyl-8-aza-bicyclo[3.2.1]octane-2β-carboxylic acid methyl ester) is a cocaine derivative belonging to a new generation of highly selective dopamine transporter (DAT) ligands (K_{D} : 9 nM and IC50 > 1000 nM for the serotonine and norepinephrine transporters).

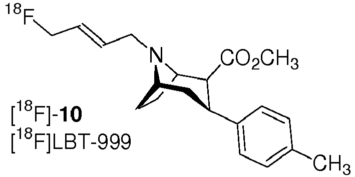

Recently, LBT-999 has been successfully labelled with the positron-emitter carbon-11 (half-life: 20.38 min) at its methyl ester function from the corresponding carboxylic acid precursor and the methylation reagent [¹¹C]methyl triflate (Dollé F. et al., 2006), permitting preliminary *in vivo* pharmacological evaluation of this radiotracer by both biodistributions in rodents and brain imaging in non-human primates with positron emission tomography (PET) (Chalon S. et al., 2006). Taken together, the results so far obtained demonstrate that LBT-999 is a highly promising candidate for *in vivo* exploration in humans of DAT, using PET (Chalon S. et al., 2006).

The chemical structure of LBT-999 permits also its labelling at its fluoromethylvinyl moiety with fluorine-18 (half-life: 109.8 min), today one of the most attractive positron-emitting radioisotopes for radiopharmaceutical chemistry.

**Table 2**

| | | | | |
|---|---|---|---|---|
| 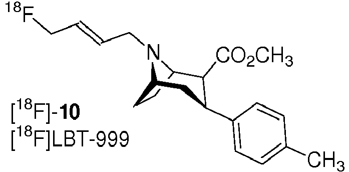 | 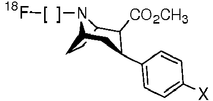 | | | |
| | | X | [ ] | |
| | A | Cl | -CH₂CH₂- | [¹⁸F]FECNT |
| | B | F | -CH₂CH₂- | β-[¹⁸F]CFT-FE |
| | C | 3'-furyl | -CH₂CH₂- | [¹⁸F]FE-3FPT |
| | D | I | -CH₂CH₂CH₂- | β-[¹⁸F]CIT-FP |
| | E | Me | -CH₂CH₂CH₂- | β-[¹⁸F]CMT-FP |
| | F | Br | -CH₂CH₂CH₂- | β-[¹⁸F]CBT-FP |
| | G | F | -CH₂CH₂CH₂- | β-[¹⁸F]CFT-FP |
| | H | Cl | -CH₂(CH=CH)CH₂- | (E/Z)-[¹⁸F]FBCINT |

The robust and reliable pathway frequently proposed for the labelling with fluorine-18 of such molecules is a two-step radiochemical process, involving first the preparation of an appropriate halogeno or better sulphonyloxy [¹⁸F]reagent. The radiosynthesis of n-bromo-, n-tosyloxy- and n-mesyloxy 1-[¹⁸F]fluoroalkanes (n = 1-3) from the corresponding bifunctional alkanes by nucleophilic aliphatic substitution with no-carrier-added [¹⁸F]fluoride as its activated K[¹⁸F]F-Kryptofix^{®}222 complex (Coenen H.H. et al., 1986; Hamacher K., et al., 1986) has been extensively studied (Block D. et al., 1987). This methodology has been applied with success to the preparation of several [¹⁸ F]fluoroethyl- (Goodman M.M. et al., 1994; Firnau G. et al., 1995; Goodman M.M. et al., 2000; Deterding T.A. et al., 2001; Votaw J.R. et al., 2002; Wilcox K.M. et al., 2002; Davis M.R. et al., 2003; Votaw J.R. et al., 2003; Votaw J.R. et al., 2004; Lindsey K.P. et al., 2004; Harada N. et al., 2004; Stehouwer J.S. et al., 2005) and [¹⁸F]fluoropropyl- (Goodman M.M. et al., 1994; Firnau G. et al., 1995; Chaly T. et al., 1996; Goodman M.M. et al., 1997; Kazumata K. et al., 1998; Ma Y. et al., 2002; Chaly T. et al., 2004) tropane derivatives (compound A-G, Table 2), like for example [¹⁸F]FECNT and β-[¹⁸F]CIT-FP. More recently, the radiosynthesis of both (*E*)- and (*Z*)-1-[¹⁸F]fluoro-4-tosyloxybut-2-ene has also been briefly described and used for the first time for the preparation of (*E*)-and (*Z*)-[¹⁸F]FBCINT (compound H, Table 2) (Chen P. et al., 1999 ; Goodman M.M. et al., 2002).

It has now been discovered that a class of compounds, referred to herein as compounds of formula (II), are useful agents for preparing the [¹⁸F]radioactively labelled compounds of formula (I). More specifically, these compounds allow to obtain the radiolabelled compounds of formula (I) in only one-step radiochemical process, with high radiolabelling yields and high radiochemical purity.

The one-step radiochemical process referred herein also brings the following advantages, when compared to the classical two-step process described above. It first allows to use only one precursor for labelling (compounds of formula II) instead of two precursors for labelling. Further, these precursors of formula (II) may be used in limited amounts (typically 2-5 mg) as compared to the two-step radiochemical process disclosed in the prior art which generally both requires a large excess and high amounts (typically ≥ 20 mg) of the nortropamine derivative. Therefore, it also reduces the level of impurities in the radiolabelled compounds of formula (I). This one-step process also makes the synthesis of compounds of formula (I) easier and faster, allowing a more robust automation. This one-step process also makes the synthesis of compounds of formula (I) safer by reducing notably the exposure to the radioactivity.

Further, this one-step process allows to prepare [¹⁸F]radiolabelled compounds of formula (I) with an higher radioactivity level, which is particularly useful for PET imaging.

Thus, in one aspect, the invention is directed to the compounds of formula (II): 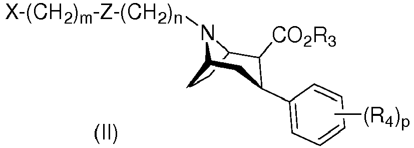 wherein:
- X is Cl, Br, I, or a group OSO₂R₅;
- Z is R₁C=CR₂ or C ≡ C ;
- R₁, R₂ are each independently H, C₁-C₆ alkyl, C₆-C₁₀ aryl, wherein said alkyl or aryl groups are optionally substituted by one to three R₆ groups;
- R₃ is H or C₁-C₆ alkyl, wherein said alkyl group is optionally substituted by one to three R₆ groups;
- p is 1, 2 or 3 ;
- R₄ is/are independently H, Cl, Br, I, F, or C₁-C₆ alkyl, wherein said alkyl group is/are optionally substituted by one to three R₆ groups;
- R₅ is a C₁-C₆ alkyl, preferably C₁-C₄ alkyl, said alkyl being optionally mono-, poly- or perhalogenated, a C₃-C₈ cycloalkyl or a C₆-C₁₂ aryl, wherein said alkyl, cycloalkyl and aryl are optionally substituted by one to three C₁-C₄ alkyl groups or F, Cl, Br, NO₂, or CN;
- R₆ is OH, F, Cl, Br, phenyl or methyl,
- m is 1, 2, 3 or 4, and
- n is 1, 2, 3 or 4,
and its stereoisomeric forms, mixtures of stereoisomeric forms, and salt forms thereof.

The leaving group X may be a OSO₂R₅ group, preferably selected from OSO₂-C₆H₄-CH₃, OSO₂-C₆H₄-Br, OSO₂-C₆H₄-NO₂, OSO₂-CH₃, OSO₂-CF₃, OSO₂-C₄F₉ or OSO₂-CH₂-CF₃. Of these, OSO₂-CgH₄-CH₃, OSO₂-CF₃, OSO₂-CH₃, commonly designates as tolyloxy (OTs), mesyloxy (OMs) and trifyloxy (OTf) respectively, are particularly preferred.

Preferably, X is Cl, Br, I, more preferably X is Cl.

Preferably, Z is R₁C=CR₂.

Advantageously, when Z is *trans* R₁C=CR₂ or C≡C, the leaving group X does not undergo an intramolecular nucleophilic substitution by the nitrogen atom of the tropane. These compounds of formula (II) are thus relatively stable under normal conditions of temperature and pressure.

Preferably, R₁ and R₂ are independently selected from H and C₁-C₄ alkyl, notably methyl or ethyl. More preferably, R₁ and R₂ are H.

Preferably, Z is *trans (E).*

Preferably, m is 1.

Preferably, n is 1.

In a preferred embodiment of the invention, the invention relates to compounds of formula (IIa): 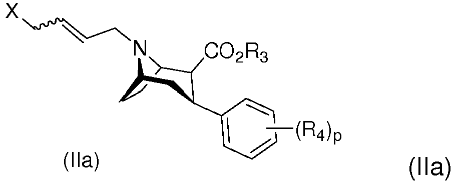 wherein X, R₃, R₄ and p are as defined above.

Preferably, R₃ is C₁-C₄ alkyl, more preferably methyl, ethyl or isopropyl, most preferably methyl.

R₄ may be C₁-C₆ alkyl, more preferably C₁-C₄ alkyl, notably a methyl group.

Preferably, p is 1 and R₄ is located in the para position on the phenyl ring.

Preferably, the compound of formula (IIa) is the N-(*E*-4-chloro-but-2-enyl)-3β-(4-methylphenyl)nortropane-2β-carboxylic methyl ester.

In another aspect, the invention also relates to a method for preparing a [¹⁸F]radiolabelled compound of formula (I): 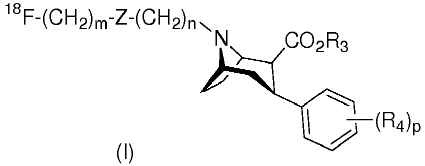 wherein, Z, R₃, R₄, m, n and p are as defined above, comprising the steps of:
i) reacting a compound of formula (III)

   X-(CH₂)ₘ-Z-(CH₂)ₙ-Y (III)

   wherein Y is Cl, Br, I or OSO₂R₅, X, Z, R₅, m and n being as defined above, with a compound of formula (IV) 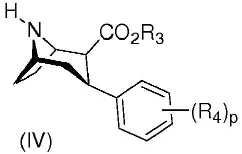 wherein R₃, R₄ and p are as defined above,
   in the presence of a base in an appropriate solvent; and optionally
ii) recovering the obtained compound of formula (II) as defined above,
iii) contacting a compound of formula (II), with a source of ¹⁸F anion in an appropriate solvent ; and optionally
iv) recovering the obtained [¹⁸F]radiolabelled compound of formula (I).

There is no particular restriction on the nature of the sources of ¹⁸F anions to be used in this reaction, and any sources of ¹⁸F anions conventionally used in reactions of this type may equally be used here, provided that it has no adverse effect on other parts of the molecule.

Examples of suitable sources of ¹⁸F anions include: alkali metal [¹⁸F]fluorides, such as sodium [¹⁸F]fluoride, potassium [¹⁸F]fluoride, cesium [¹⁸F]fluoride; ammonium [¹⁸F]fluoride, tetraalkylammonium [¹⁸F]fluorides, such as tetrabutylammonium [¹⁸F]fluoride. Of these, the alkali metal [¹⁸F]fluorides, and notably a potassium fluoride, are preferred.

The source of ¹⁸F anions may be activated by the presence of a ligand able to complex the counter cationic species of the source of ¹⁸F anions. The ligand may be notably a cyclic or polycyclic multidentate ligand.

Examples of suitable ligands include notably crown ethers such as 1,4,7,10,13-pentaoxacyclooctadecane (18C6) or cryptands such as 4,7,13,16, 21,24-hexaoxa-1,10-diazabicyclo-[8,8,8]hexacosane sold under the name K222^{®}.

Preferably, the source of ¹⁸F anions is an alkaline metal [¹⁸F]fluoride-cryptate complex, notably a potassium [¹⁸F]fluoride-cryptate complex, preferably the potassium [¹⁸F]fluoride - 4,7,13,16,21,24-hexaoxa-1,10-diazabicyclo-[8,8,8]hexacosane (K[¹⁸F]/K222).

The complex K[¹⁸F]/K222^{®} may be prepared by any conventional methods, for example as disclosed in F. Dollé et al., 1999; L. Dolci et al., 1999.

The method for preparing the [¹⁸F]radiolabelled compound of formula (I) can take place over a wide range of temperatures, and the precise reaction temperature is not critical to the invention. In general, it is convenient to carry out the reaction at a temperature of from about 50°C to about 150°C (more preferably, from about 80°C to about 100°C).

The time required for the reaction may also vary widely, depending on many factors, notably the reaction temperature and the nature of the reagents. However, provided that the reaction is effected under the preferred conditions outlined above, a period of from about 5 minutes to about 15 minutes will usually suffice.

The compound thus prepared may be recovered from the reaction mixture by conventional means. For example, the compounds may be recovered by filtration on a pre-packed separation column.

Additionally, the product can, if desired, be further purified by various well known techniques, such as chromatography techniques, notably High Performance Liquid Chromatography (HPLC).

There is no particular restriction on the nature of the base to be used in this reaction, and any base conventionally used in reactions of this type may equally be used here, provided that it has no adverse effect on other parts of the molecule. Examples of suitable bases include alkylamines, such as triethylamine.

There is no particular restriction on the nature of the solvent to be employed, provided that it has no adverse effect on the reaction or on the reagents involved. Examples of appropriate solvents include notably polar solvents such as acetonitrile.

In a still further aspect, the invention relates to a compound of formula (IIIa): 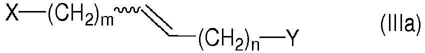 wherein X is Cl, Br, I and Y is OSO₂R₅, R₅, m and n being as defined above.

Preferably, m and n are 1.

Preferably, the stereochemistry of (IIIa) is *trans.*

Preferably, Y is OSO₂(C₆H₄)CH₃ (tolyloxy), notably p-tolyloxy.

Preferably, X is Cl.

Preferably, the compound of formula (IIIa) is the *E*-1-chloro-4-tosyloxy-2-butene.

Compounds of formula (IIIa) useful according to the invention may be prepared by the application or adaptation of known methods, by which is meant methods used heretofore or described in the literature, for example those described by R. C. Larock in Comprehensive Organic Transformations, VCH Publishers, 1989.

The invention also relates to the use of a compound of formula (II) as defined above for preparing a compound of formula (I) as defined above.

In a further aspect, the invention also relates to a kit for the preparation of a [¹⁸F]radioactively labelled compound of formula (I), comprising:
(a) a compound of formula (II), as defined above ; and
(b) a source of ¹⁸F anions.

Optionally, the kit can include items of apparatus, such as a reaction vessel, device for transferring isotopic material to the reaction vessel, pre-packed separation column for separating product from excess reactants, shielding and the like, as known in the art.

### Definitions

The following terms and expressions contained herein are defined as follows:
As used herein, the term "alkyl" refers to a straight-chain, or branched alkyl group having 1 to 6 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, etc. Preferred alkyl groups are those which contain 1 to 4 carbons. A designation such as "C₁-C₄ alkyl" refers to an alkyl radical containing from 1 to 4 carbon atoms.

As used herein, the term "cycloalkyl" refers to a saturated or partially saturated mono- or bicyclic alkyl ring system containing 3 to 8 carbon atoms. A designation such as "C₅-C₇ cycloalkyl" refers to a cycloalkyl radical containing from 5 to 7 ring carbon atoms. Preferred cycloalkyl groups include those containing 5 or 6 ring carbon atoms. Examples of cycloalkyl groups include such groups as cyclopropyl, cyclobutyl, cyclopentyl, cyclohexyl, cycloheptyl, cyclooctyl.

As used herein, the term "aryl" refers to a substituted or unsubstituted, mono- or bicyclic hydrocarbon aromatic ring system having 6 to 12 ring carbon atoms. Examples include phenyl and naphthyl. Preferred aryl groups include unsubstituted or substituted phenyl and naphthyl groups.

As used herein, "stable compound" or "stable structure" refers to a compound that is sufficiently robust to survive isolation to a useful degree of purity from a reaction mixture.

As used herein, "stereoisomeric forms" refers notably to the *cis* and *trans* isomeric forms.

The starting materials and intermediates of compounds useful according to the invention may be prepared by the application or adaptation of known methods, for example methods as described in the Examples or their obvious chemical equivalents.

The following examples are presented as an illustration only.

### EXAMPLES

### 1. N-(E-4-chloro-but-2-enyl)-3β-(4-methylphenyl)nortropane-2β-carboxylic methyl ester (9)

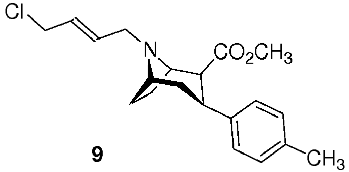

The strategy of synthesis was the reaction between the 3β-(4-methylphenyl)-nortropane-2β-carboxylic methyl ester (**4**) and a sulphonate derivative.

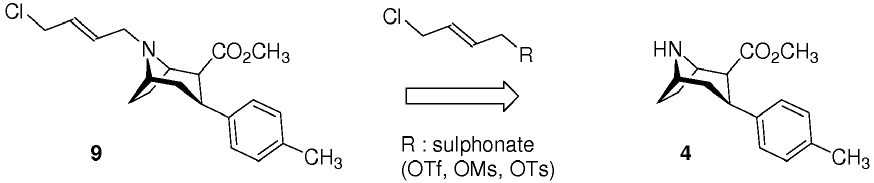

### a) 2β-carbomethoxy-3β-(4-methylphenyl)nortropane (4)

The 2β-carbomethoxy-3β-(4-methylphenyl)nortropane (**4**) has been synthesized according to the procedures already published : 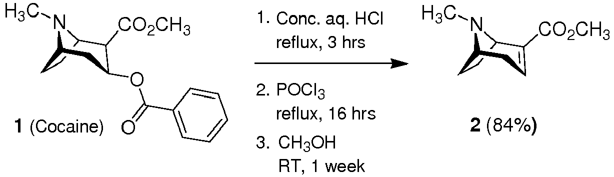 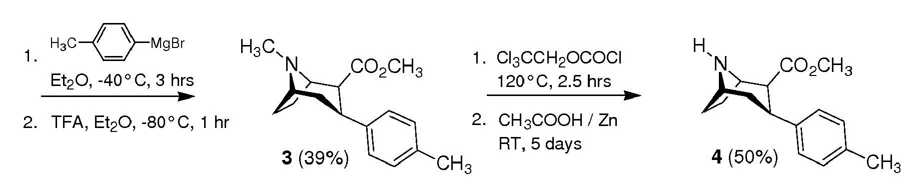 Milius R.A. et al, J. Med. Chem., 1991, 34, 1728-1731 .
Blough B.E. et al, J. Med. Chem., 1996, 39, 4027-4035.
Emond P. et al, J. Med. Chem., 1997, 40, 1366-1371.

### b) N-(E-4-chloro-but-2-enyl)-3β-(4-methylphenyl)-nortropane-2β-carboxylic methyl ester (9)

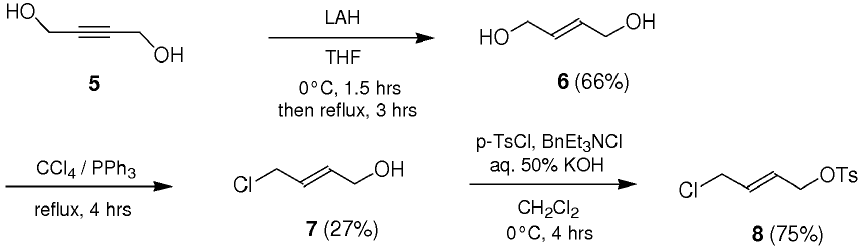

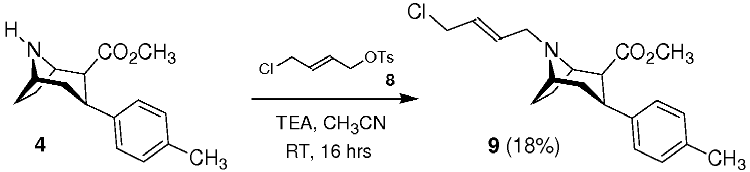

The 2-butyne-1,4-diol (**5**) was reduced to the *E*-2-butene-1,4-diol (**6**) by LiAlH₄ according to the procedure described by Kayaleh et al., 2000). The *E*-2-butene-1,4-diol **(6)** was refluxed in CCl₄ in presence of triphenylphosphine to give the mono chlorinated derivative **(7)** which was transformed to the tosylate derivative (**8**). This compound reacted with the nortropane derivative (**4**) to give the chloro derivative (**9**).

### Experimental Section

### E-2-butene-1,4-diol (6)

A solution of LAH (300 mmoles / 400 mL) was cooled to 0°C under nitrogen atmosphere. A solution of 2-butyne-1,4-diol (**5**) (21.5 g, 250 mmoles, 1 eq) in 200 mL of anhydrous THF was added dropwise over 90 min maintaining the temperature under 0°C. Following complete addition of (**5**), the reaction mixture was refluxed for 3 hours. 3M NaOH aq. solution was added slowly at 0°C until no gas evolution was observed. The solution was filtrated. The precipitate was washed by THF (3 x 100 mL). The organic layers were combined, dried on MgSO₄ and the solvent was removed under reduced pressure to give the crude product (**6**). 14.5 g (yield 66%).
¹H NMR (D₂O) : 3.94 (broad s, 4H), 4.67 (broad s, 2H), 5.69 (broad s, 2H).
¹³C NMR (D₂O) : 61.63, 129.77.

### E-1-chloro-4-ol-2-butene (7)

10 g (114 mmoles) of E-2-butene-1,4-diol **(6)** and 33.3 g (127 mmoles, 1.1 eq) of triphenylphosphines were stirred vigorously in CCl₄ (100 mL) and refluxed for 4 hours. CH₂Cl₂ (200 mL) was added and the organic layer was washed with H₂O (2 x 100 mL), dried on MgSO₄ and the solvent was removed under reduced pressure to give the crude product (**7**) which was purified by silica gel column chromatography (CH₂Cl₂). 3.3 g (yield 27%).
¹H NMR (CDCl₃) : 4.06 (dd, J³ = 4.84 Hz, J⁴ = 0.97 Hz, 2H), 4.06 (d, J³ = 3.55 Hz, 2H), 5.69 (broad s, 2H), 5.88 (m, 2H).
¹³C NMR (CDCl₃) : 44.45, 62.49, 126.95, 133.84.

### E-1-chloro-4-tosyloxy-2-butene (8)

2 g (18.8 mmoles) of *E*-1-chloro-4-ol-2-butene (**7**) and 220 mg (0.94 mmoles, 0.05 eq) of benzyltriethylammonium chloride were placed in CH₂Cl₂ (40 mL) and KOH aq. 50% (40 mL) and the mixture was cooled to 0°C. A solution of p-toluenesulfonyl chloride (3.93 g, 20.7 mmoles, 1.1 eq) in CH₂Cl₂ (40 mL) was added dropwise maintaining the temperature under 0°C. The reaction mixture was stirred 4 hours at 0°C, and dluted with H₂O (40 mL). Aqueous layer was extracted by CH₂Cl₂ (2 x 80 mL). The organic layers were combined, washed with brine and dried over MgSO₄ and the solvent was evaporated under reduced pressure to afford the crude product (**8**) which was used without purification. 3.43 g (yield 75%).
¹H NMR (CDCl₃) : 2.45 (s, 3H), 3.99 (d, J³ = 5.6 Hz, 2H), 4.55 (d, J³ = 5.1 Hz, 2H), 5.82(m, 2H), 7.79 (d, J³ = 8.2 Hz), 7.79 (d, J³ = 8.2 Hz, 2H).
¹³C NMR (CDCl₃) : 21.72, 43.46, 69.37, 126.36, 127.99, 130.06, 131.66.

### N-(E-4-chloro-but-2-enyl)-3β-(4-methylphenyl)nortropane-2β-carboxylic methyl ester (9)

1.5 g (5.8 mmoles, 1.5 eq) of E-1-chloro-4-tosyloxy-2-butene (**8**) and 800 µL (5.8 mmoles, 1.5 eq) of triethylamine in 10 mL of CH₃CN were cooled to 0°C. A solution of 3β-(4-methylphenyl)nortropane-2β-carboxylic acid methyl ester (**4**) in (1 g, 1 eq) CH₃CN (20 mL) was added dropwise maintaining temperature under 0°C. Then the reaction mixture was stirred at room temperature overnight. CH₃CN was evaporated under reduced pressure and the crude product was purified by alumina gel column chromatography (Pentane/CH₂Cl₂ 50/50 to 0/100). 3.3 g (yield 18%).
¹H NMR (CDCl₃) : 1.69 (m, 3H), 2.03 (m, 2H), 2.29 (s, 3H), 2.60 (td, J³ = 12.6 Hz, J³ = 2.6 Hz 1 H), 2.94 (m, 4H), 3.41 (m, 1 H), 3.51 (s, 3H), 3.66 (1 H), 4.05 (d, J³ = 3.12 Hz, 2H), 5.75 (m, 2H), 7.08 (d, J³ = 8.2 Hz, 2H), 7.16 (d, J³ = 8.2 Hz, 2H).
¹³C NMR (CDCl₃) : 21.06, 26.02, 26.14, 33.95, 34.23, 44.88, 51.09, 52.88, 54.92, 61.46, 62.44, 127.31, 127.59, 128.75, 133.69, 135.31, 139.97, 172.07.
ES-MS (MH⁺) calculated : 348.17 found : 348.32.

### 2. 8-((E)-4-[¹⁸F]fluoro-but-2-enyl)-3β-p-tolyl-8-aza-bicyclo-[3.2.1]octane-2β-carboxylic acid methyl ester ([¹⁸F]-10, [¹⁸F]LBT-999)

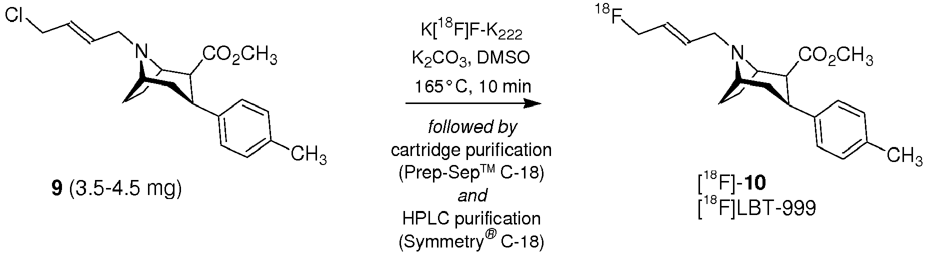

The one-step radiosynthesis of [¹⁸F]LBT-999 ([¹⁸F]-**10**) is hereabove.

Introduction of the cyclotron-produced fluorine-18 as the, no-carrier-added, activated K[¹⁸F]F-Kryptofix^{®}222 complex (Coenen H.H. et al., 1986 ; Hamacher K. et al., 1986) was performed as follows : a DMSO solution (600 µL) containing 3.5-4.5 mg of 8-((*E*)-4-Chloro-but-2-enyl)-3β-*p*-tolyl-8-aza-bicyclo-[3.2.1]octane-2β-carboxylic acid methyl ester (**9**, 10.0-12.9 µmol) were transferred (See experimental part) to the dried K[¹⁸F]F-K₂₂₂ complex in a reaction vial (Vacutainer^{®} tube). The unsealed tube was thoroughly vortexed and then heated (heating block) at 165°C for 10 minutes without stirring the contents. The reaction vessel was cooled (ice-water bath) and the remaining radioactivity was measured, which was 96% to over 99% of the initial radioactivity. The radiochemical yields of fluorine-18 incorporation, calculated from the TLC-radiochromatogram and defined as the ratio of radioactivity area of [¹⁸F]-**10** over total fluorine-18 radioactivity area, were about 30% to 40%. At this stage, a C-18 Sep-Pak cartridge (PrepSep^{™} R-C18 Extraction Column, see experimental) was used to rapidly isolate [¹⁸F]-**10** (with a radiochemical purity >90%, according to radio-HPLC) from the reaction, which represented 30-35% of the total radioactivity amount engaged in the fluorination process whereas most likely non reacted [¹⁸F]fluoride, 65-70% of the total radioactivity, was eluted off the cartridge (waste). These values clearly confirmed the radiochemical yields measured by radio-TLC.

HPLC purification of [¹⁸F]LBT-999 ([¹⁸F]-**10**) was performed on a semipreparative Symmetry^{®} C18 column (HPLC B, see experimental), using a mixture of H₂O/CH₃CN/TFA (72:28:0.1 (v/v/v)) as the eluent. Using these conditions, [¹⁸F]-**10** (*t*_{R} : 12.5-13.5 minutes) could be obtained with a >95% chemically and radiochemically purity and was ideally separated within a short total purification time (less than 20 minutes) from the non-labelled chloro-derivative **9** (*t*_{R} : 15.5-17.5 minutes), as well as from an unidentified fluorine-18-labelled impurity at *t*_{R} : 14.0-15.0 minutes (up to 10% compared to [¹⁸F]-**10**). A too low chemical purity (< 90%) was observed using a previously reported HPLC system used for the purification of [¹⁸F]-**10** (HPLC C, column: semipreparative SunFire^{™} C18, eluant : H₂O/CH₃CN/TFA : 70:30:0.1 (v/v/v), *t*_{R} ([¹⁸F]-**10**): 9.0-10.0 min (Dollé F. et al., 2006).

Introduction of fluorine-18 was also performed with the K[¹⁸F]F-Kryptofix^{®}222 complex and microwave activation (at 100 Watt for 2 minutes). Incorporation yields (calculated from the TLC-radiochromatogram or after the C-18 Sep-Pak cartridge purification) were similar to those described above, with however formation in moderately higher ratio of the unidentified fluorine-18-labelled impurity (about 40-50% compared to [¹⁸F]-**10**). HPLC purification using the system described above gave nevertheless >95% chemically and radiochemically pure [¹⁸F]-**10** (*t*_{R} : 12.5-13.5 minutes), still well separated from this side product (*t*_{R} : 14.0-15.0 minutes).

Formulation of [¹⁸F]LBT-999 ([¹⁸F]-**10**) as *i.v.* injectable solution was performed using a home-made Sep-pak^{®}Plus C18 device. The HPLC-collected fraction containing the radiotracer was diluted with water and the resulting solution was passed through a C18 Sep-pak^{®} cartridge. The cartridge was then washed with water, partially dried with nitrogen and finally eluted with ethanol followed by physiological saline. The solution was then sterile-filtered and diluted with physiological saline to an ethanol concentration below 10%.

Quality controls of [¹⁸F]LBT-999 ([¹⁸F]-**10**) were performed on an aliquot of the preparation ready for *i*.*v*. injection. The radiotracer preparation was a clear and colourless solution with a measured pH between 5 and 7. As demonstrated by analytical HPLC analysis (HPLC D, see experimental), the radiotracer preparation was found to be > 95% chemically and radiochemically pure (**1**, *t*_{R} : 2.25 min). The preparation was shown to be free of the non-radioactive precursor **9** (*t*_{R} : 3.45 min) as well as the unidentified fluorine-18-labelled side-product (*t*_{R} : 2.58 min), and was chemically and radiochemically stable for at least 120 minutes.

### Conclusion

The highly selective dopamine transporter ligand LBT-999 (**10**, 8-((*E*)-4-fluoro-but-2-enyl)-3β-*p*-tolyl-8-aza-bicyclo[3.2.1]octane-2β-carboxylic acid methyl ester) has been labelled in one radiochemical step with fluorine-18 at its fluoromethylvinyl moiety. Typically, 70-100 mCi of [¹⁸F]LBT-999 ([¹⁸F]-**10**, 2.59-3.07 GBq, > 95% chemically and radiochemically pure) could be obtained with a specific radioactivity of 1-3 Ci/µmol (37-111 GBq/µmol) in 80-85 minutes of radiosynthesis (HPLC purification and formulation included), starting from a 1000 mCi (37.0 GBq) [¹⁸F]fluoride production batch (overall yields : 7.0% to 10.0% non decay-corrected and 11.6-17.1% decay-corrected).

### Experimental

### General

### Chemicals, Flash chromatography and TLC analysis.

Chemicals were purchased from Aldrich-, Fluka- or Sigma France and were used without further purification. Flash chromatographies were conducted on silica gel (0.63-0.200 mm, VWR) columns. TLCs were run on pre-coated plates of silica gel 60F254 (Merck). The compounds were localized (1) when possible at 254 nm using a UV-lamp and/or (2) by dipping the TLC-plates in a 1% ethanolic ninhydrin solution and heating on a hot plate. Radioactive spots were detected using a Berthold TraceMaster 20 automatic TLC linear analyser.

### HPLC analysis

[HPLC A]: Equipment: system equipped with a Waters 600 Pump and Waters 600 Controller, a Shimadzu SPD10-AVP UV-multi-wavelength detector and a miniature ionisation chamber probe ; column: semipreparative X-Terra® C18, Waters (300 x 7.8 mm) ; porosity: 7 µm; eluent H₂O / CH₃CN / TFA : 60:40:0.1 (v/v/v); flow rate: 4 mL/min; temperature: RT ; absorbance detection at λ = 220 nm.

[HPLC B]: Equipment: system equipped with a Waters 600 Pump and Waters 600 Controller, a Shimadzu SPD10-AVP UV-multi-wavelength detector and a miniature ionisation chamber probe ; column: semipreparative Symmetry® C18, Waters (300 x 7.8 mm) ; porosity: 7 µm; eluent H₂O / CH₃CN / TFA : 72:28:0.1 (v/v/v); flow rate: 5 mL/min; temperature: RT ; absorbance detection at λ = 220 nm.

[HPLC C]: Equipment: system equipped with a Waters 600 Pump and Waters 600 Controller, a Shimadzu SPD10-AVP UV-multi-wavelength detector and a miniature ionisation chamber probe ; column: semipreparative SunFireTM C18, Waters (250 x 10.0 mm) ; porosity: 5 µm; eluent H₂O / CH₃CN / TFA : 70:30:0.1 (v/v/v); flow rate: 5 mL/min; temperature: RT ; absorbance detection at λ = 220 nm.

[HPLC D]: Equipment: Waters Alliance 2690 (or a Waters binary HPLC pump 1525) equipped with a UV spectrophotometer (Photodiode Array Detector, Waters 996) and a Berthold LB509 radioactivity detector; column: analytical Symmetry-M® C-18, Waters (50 x 4.6 mm); porosity: 5.0 µm; conditions: isocratic elution with solvA / solvB: 55 / 45 (v/v) [solvent A: H₂O containing Low-UV PIC® B7 reagent (composition (% by weight) : methanol (18-22%), heptane sulfonic acid - sodium salts (4-6%), phosphate buffer solution (3-7%), water (65-75%), pH 3, Waters), 20 mL for 1000 mL; solvent B: H₂O / CH₃CN: 30:70 (v/v) containing Low-UV PIC® B7 reagent (20 mL for 1000 mL)]; flow rate: 2.0 mL/min; temperature: 30°C; UV detection atλ: 205 nm.

### Radioisotope production

No-carrier-added aqueous [¹⁸F]fluoride ion was produced via the [18O(p,n)¹⁸F] nuclear reaction by irradiation of a 2 mL [¹⁸O]water (> 97%-enriched, CortecNet, Paris, France) target on a IBA Cyclone-18/9 cyclotron (18 MeV proton beam) and was transferred to the appropriate hot cell. Target hardware: commercial, 2-mL, two-port, stainless steel target holder equipped with a domed-end niobium cylinder insert. Target to hot cell liquid-transfer system : 60 m PTFE line (0.8 mm internal diameter ; 1/16 inch external diameter), 2.0 bar He drive pressure, transfer time 3-6 min. Typical production of [¹⁸F]Fluoride at the end of bombardment for a 20 µA, 30 min (10 µA.h) irradiation: 750-800 mCi (27.7-29.6 GBq).

### Miscellaneous

Radiosyntheses using fluorine-18, including the HPLC purifications, were performed in a 7.5-cm-lead shielded cell using a computer assisted Zymate robot system (Zymark corporation, USA). Microwave activation was performed with a MicroWell 10 oven (2.45 GHz), Labwell AB, Sweden.

### Radiochemistry

### Preparation of the K[¹⁸F]F-K₂₂₂-complex

In order to recover and recycle the [¹⁸O]water target, the 2 mL of aqueous [¹⁸F]fluoride from the target holder were passed through an anion exchange resin (Sep-pak^{®} Light Waters Accell^{™} Plus QMA cartridge (initially in the chloride form, then washed with aq. 1M NaHCO₃ (2 mL) and rinsed with water (20 mL) and CH₃CN (10 mL)) by helium pressure (1.5-2.0 bar). Helium was blown through the column to maximally extract the last traces of [¹⁸O]water. The [¹⁸F]fluoride ion was then eluted from the resin, using an aq. K₂CO₃ solution (1.0 mL of a 1.0 mg/mL solution), into a Vacutainer^{®} tube containing Kryptofix^{®}222 (K₂₂₂: 4,7,13,16,21,24-hexaoxa-1,10-diazabicyclo[8.8.8] hexacosane, 12.0 to 15.0 mg). The resulting solution was then gently concentrated to dryness at 145-150°C under a nitrogen stream for 10 min to give no-carrier-added K[¹⁸F]F-K₂₂₂ complex as a white semi-solid residue.

### Preparation of 8-((E)-4-[¹⁸Flfluoro-but-2-enyl)-3β-p-tolyl-8-aza-bicyclo-[3.2.1]octane-2β-carboxylic acid methyl ester ([¹⁸F]-10, [¹⁸F]LBT-999).

### Zymate XP (Zymark)

A. Procedure using conventional heating. DMSO (600 µL) containing 8-((*E*)-4-Chloro-but-2-enyl)-3β-*p*-tolyl-8-aza-bicyclo[3.2.1]octane-2β-carboxylic acid methyl ester (**9**, 3.5-4.5 mg, 10.0-12.9 µmol) were directly added into the Vacutainer^{®} tube containing the dried K[¹⁸F]F-K₂₂₂ complex. The tube (not sealed) was thoroughly vortexed (30 s) and then placed in a heating block (at 90°C, for 10 min) without stirring the contents. The reaction vessel was then cooled using an ice-water bath, the remaining radioactivity measured (which was 96% to over 99% of the initial radioactivity) and the reaction mixture analyzed by radio-TLC. The reaction yield was calculated from the TLC-radiochromatogram and defined as the ratio of radioactivity area of [¹⁸F]LBT-999 ([¹⁸F]-**10**) over total fluorine-18 radioactivity area (SiO₂-TLC (EtOAc) : *R_{f}* : [¹⁸F]-**10** : 0.5 and *R_{f}* : [¹⁸F]fluoride ion : 0.0). The reaction mixture was diluted with water (1 mL) and transferred onto a C18 Sep-Pak cartridge (PrepSep^{™} R-C18 Extraction Column, Fisher Scientific, activated beforehand with EtOH (2 mL) and then rinsed with water (10 mL)), pre-filled with water (2 mL). The tube was rinsed twice with water (1 mL), which was also transferred and added to the diluted reaction mixture on top of the cartridge (0.5-1.5% of the total radioactivity amount engaged in the fluorination process was lost in the initial tube). An additional portion of water (2 mL) was further added to the diluted reaction mixture on top of the cartridge. The whole was then passed through the cartridge, which was then washed with water (3 mL) and partially dried for 0.5 min by applying a nitrogen stream. [¹⁸F]-**10** was eluted from the cartridge with CH₂Cl₂ (3 mL) into an empty 5 mL reaction vial. Twice 1 mL of CH₂Cl₂ was used to wash the cartridge (only 1-2% was left and lost on the cartridge) for maximal transfer of [¹⁸F]-**10**. 30-35% of the total radioactivity amount engaged in the fluorination process was eluted with CH₂Cl₂ and was analysed by radio-HPLC (HPLC A). The incorporation yield was estimated after the C18 Sep-pak cartridge elution by the CH₂Cl₂- over total eluted radioactivity (DMSO/H₂O + CH₂Cl₂) ratio. The resulting CH₂Cl₂ solution was concentrated to dryness (at 65-75°C under a gentle nitrogen stream for 3-5 min). Finally, the above residue was redissolved in the HPLC solvent used for purification (1.0 mL) and the crude was injected onto HPLC (HPLC B). Isocratic elution gave pure [¹⁸F]-**10** (*t*_{R}: 12.5-13.5 min), well separated from unlabelled **9** (*t*_{R} : 15.5-17.5 min) and an unidentified fluorine-18-labelled impurity [¹⁸F]-**11** (*t*_{R} : 14.0-15.0 min).
B. Procedure using microwave activation. Same as procedure A, but instead of the heating block a dedicated microwave oven (at 100 Watt, for 2 min) was used.

Formulation of [¹⁸F]LBT-999 ([¹⁸F]-**10**). Formulation of the labelled product for i.v. injection was effected as follows: The HPLC-collected fraction containing the radiotracer was diluted with water (50 mL). The resulting solution was passed through a Sep-pak^{®}Plus C18 cartridge (Waters, washed with 2 mL of EtOH and then rinsed with 10 mL of water prior to use). The cartridge was washed with water (10 mL) and partially dried by applying a nitrogen stream for 10 s. The radiotracer was eluted with 2 mL of EtOH (less than 10% of the total radioactivity was left on the cartridge) followed by 8 mL of physiological saline and filtered on a 0.22 µm GS-Millipore filter (vented). Finally, physiological saline was added to lower the EtOH concentration below 10%. This whole process was performed using a remote-controlled dedicated home-made device based on a literature procedure (Lemaire C. et al., 1999).

Quality control of [¹⁸F]LBT-999 ([¹⁸F]-**10**). The radiotracer preparation was visually inspected for clarity, absence of colour and particulates. An aliquot of the preparation was removed for determination of pH using standard pH-paper. Chemical and radiochemical purities were also assessed on this aliquot by HPLC (HPLC D), with a sample of authentic **10** (*t*_{R} : 2.25 min) which synthesis and characterization were disclosed in Dollé F et al., 2006. Particular attention was paid to the absence of non-radioactive precursor **9** (t_{R}: 3.45 min). Chemical and radiochemical stability of the entire preparation was tested by HPLC (HPLC D) at regular 15-min intervals during 120 min. Specific radioactivity of the radiotracer was calculated from three consecutive HPLC (HPLC D) analyses (average) and determined as follows: The area of the UV absorbance peak corresponding to the radiolabelled product was measured (integrated) on the HPLC chromatogram and compared to a standard curve relating mass to UV absorbance.

### REFERENCES

Block D, Coenen HH, Stocklin G. J. Label. Compds Radiopharm. 1987, 24: 1029-1042.
Blough B.E. et al, J. Med. Chem., 1996, 39, 4027-4035.
Chalon S, Hall H, Saba W, Garreau L, Dollé F, Halldin C, Emond P, Bottlaender M, Deloye J-B, Helfenbein J, Madelmont J-C, Bodard S, Mincheva Z, Besnard J-C and Guilloteau D. J. Pharm. Exp. Ther. 2006, 317 (1), 147-152*.*
Chaly T, Baldwin RM, Neumeyer JL, Hellman MJ, Dhawan V, Garg PK, Tamagnan G, Staley JK, Al-Tikriti MS, Hou Y, Zoghbi SS, Gu XH, Zong R, Eidelberg D. Nucl. Med. Biol. 2004, 31:125-131.
Chaly T, Dhawan V, Kazumata K, Antonini A, Margouleff C, Dahl JR, Belakhlef A, Margouleff D, Yee A, Wang S, Tamagnan G, Neumeyer JL, Eidelberg D. Nucl. Med. Biol. 1996, 23:999-1004.
Chen P, Kilts C, Camp VM, Ely T, Keil R, Malveaux E, Votaw D, Hoffman JM, Goodman MM. Abstract of the 13th International Symposium on Radiopharmaceutical Chemistry (Saint-Louis, MO, USA, June 27 - July 1st, 1999). J. Label. Compds Radiopharm. 1999, 42:S400.
Coenen HH, Klatte B, Knoechel A, Schueller M, Stocklin G. J. Label. Compds Radiopharm. 1986, 23: 455-467.
Davis MR, Votaw JR, Bremner JD, Byas-Smith MG, Faber TL, Voll RJ, Hoffman JM, Grafton ST, Kilts CD, Goodman MM. J. Nucl. Med. 2003, 44: 855-861.
Deterding TA, Votaw JR, Wang CK, Eshima D, Eshima L, Keil R, Malveaux E, Kilts CD, Goodman MM, Hoffman JM. J. Nucl. Med. 2001, 42:376-381.
Dolci L, Dollé F, Valette H, Vaufrey F, Fuseau C, Bottlaender M and Crouzel C, Bioorg. Med. Chem. 1999, 7 (3), 467-479.
Dollé F, Emond P, Mavel S, Demphel S, Hinnen F, Mincheva Z, Saba W, Valette H, Chalon S, Halldin C, Helfenbein J, Legaillard J, Madelmont J-C, Deloye J-B, Bottlaender M, Guilloteau D. Bioorg. Med. Chem. 2006, 14:1115-1125.
Dollé F, Dolci L, Valette H, Hinnen F, Vaufrey F, Guenther I, Fuseau C, Coulon C, Bottlaender M and Crouzel C, J. Med. Chem. 1999, 42 (12), 2251-2259.
Emond P, Garreau L, Chalon S, Boazi M, Caillet M, Bricard J, Frangin Y, Mauclaire L, Besnard J-C, Guilloteau D, J. Med. Chem., 1997, 40, 1366-1371.
Firnau G, Chen JJ, Murthy D. Abstract of the 11th International Symposium on Radiopharmaceutical Chemistry (Vancouver, B.C., Canada, August 13-17, 1995). J. Label. Compds Radiopharm. 1995, 37:55-56.
Goodman MM, Chen P. Fluoroalkenyl nortropanes. WO 00064490 A1: US Patent, 2000; pp 37 pp.
Goodman MM, Kabalka GW, Kung MP, Kung HF, Meyer MA Abstract of the 10th International Symposium on Radiopharmaceutical Chemistry (Kyoto, Japan, October 25-28, 1993). J. Label. Compds Radiopharm. 1994, 34:488-490.
Goodman MM, Keil R, Shoup TM, Eshima D, Eshima L, Kilts C, Votaw J, Camp VM, Votaw D, Smith E, Kung MP, Malveaux E, Watts R, Huerkamp M, Wu D, Garcia E, Hoffman JM. J. Nucl. Med. 1997, 38:119-126.
Goodman MM, Kilts CD, Keil R, Shi B, Martarello L, Xing D, Votaw J, Ely TD, Lambert P, Owens MJ, Camp VM, Malveaux E, Hoffman JM. Nucl. Med. Biol. 2000, 27:1-12.
Hamacher K, Coenen HH, Stöcklin G. J. Nucl. Med. 1986, 27: 235-238.
Harada N, Ohba H, Fukumoto D, Kakiuchi T, Tsukada H. Synapse 2004, 54:37-45.
Kayaleh NE, Gupta RC, Johnson F, J. Org. Chem., 2000, 65, 4515-4522.
Kazumata K, Dhawan V, Chaly T, Antonini A, Margouleff C, Belakhlef A, Neumeyer J, Eidelberg D. J. Nucl. Med. 1998, 39:1521-1530.
Kilbourn MR. In Fluorine-18 Labeling of Radiopharmaceuticals, Nuclear Science Series (Kilbourn MR Ed.), National Academy Press, Washington, D.C. (1990), 1-149.
Lasne M-C, Perrio C, Rouden J, Barré L, Roeda D, Dollé F, Crouzel C. In Chemistry of β+-Emitting Compounds Based on Fluorine-18, Topics in Current Chemistry (Krause W. Ed.), Vol. 222, Springer-Verlag Berlin Heidelberg (2002), 201-258.
Lemaire C, Plenevaux A, Aerts J, Del Fiore G, Brihaye C, Le Bars D, Comar D and Luxen A. J. Label. Compds Radiopharm. 1999; 42: 63-75.
Lindsey KP, Wilcox KM, Votaw JR, Goodman MM, Plisson C, Carroll Fl, Rice KC, Howell LL. J. Pharmacol. Exp. Ther. 2004, 309:959-969.
Ma Y, Dhawan V, Mentis M, Chaly T, Spetsieris PG, Eidelberg D. Synapse 2002, 45:125-133.
Milius RA, Saha JK, Madras BK, Neumeyer JL, J. Med. Chem., 1991, 34, 1728-1731.
Stehouwer JS, Plisson C, Jarkas N, Zeng F, Voll RJ, Williams L, Martarello L, Votaw JR, Tamagnan G, Goodman MM. J. Med. Chem. 2005, 48:7080-7083.
Votaw JR, Byas-Smith MG, Hua J, Voll R, Martarello L, Levey Al, Bowman FD, Goodman MM Anesthesiology 2003, 98:404-411.
Votaw JR, Byas-Smith MG, Voll R, Halkar R, Goodman MM. Anesthesiology 2004, 101:1128-1135.
Votaw JR, Howell LL, Martarello L, Hoffman JM, Kilts CD, Lindsey KP, Goodman MM. Synapse 2002, 44,203-210.
Welch MJ, Redvanly CS. In Handbook of radiopharmaceuticals - Radiochemistry and applications (Welch MJ, Redvanly CS Ed.), New York-Chichester-Brisbane-Toronto, Wiley-Interscience Pub. (2003), 1-848.
Wilcox KM, Lindsey KP, Votaw JR, Goodman MM, Martarello L, Carroll Fl, Howell LL. Synapse 2002, 43:78-85.

## Claims

1. A compound of formula (II) 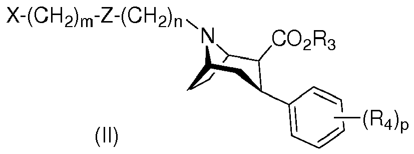 wherein:
- X is Cl, Br, I, or a group -OSO₂R₅;
- Z is R₁C=CR₂ or C ≡ C ;
- R₁, R₂ are each independently H, C₁-C₆ alkyl, C₆-C₁₀ aryl, wherein said alkyl or aryl groups are optionally substituted by one to three R₆ groups;
- R₃ is H or C₁-C₆ alkyl, wherein said alkyl group is optionally substituted by one to three R₆ groups;
- p is 1, 2 or 3 ;
- R₄ is/are independently H, Cl, Br, I, F, or C₁-C₆ alkyl, wherein said alkyl group is/are optionally substituted by one to three R₆ groups;
- R₅ is a C₁-C₆ alkyl, said alkyl being optionally mono-, poly- or perhalogenated, a C₃-C₈ cycloalkyl or a C₆-C₁₂ aryl, wherein said alkyl, cycloalkyl and aryl are optionally substituted by one to three C₁-C₄ alkyl groups or F, Cl, Br, NO₂, or CN;
- R₆ is OH, F, Cl, Br, phenyl or methyl,
- m is 1, 2, 3 or 4, and
- n is 1, 2, 3 or 4,
and its stereoisomeric forms, mixtures of stereoisomeric forms, and salt forms thereof.

2. The compound of claim 1, wherein X is Cl, Br, I.

3. The compound of claim 2, wherein X is Cl.

4. The compound of any of claims 1 to 3, wherein Z is R₁C=CR₂.

5. The compound of claim 4, wherein Z is *trans.*

6. The compound of any of claims 1 to 5, which is of formula (IIa): 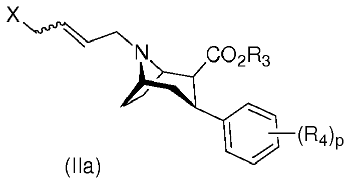 wherein X, R₃, R₄ and p are as defined in claims 1 to 3.

7. The compound of claim 6, which is the N-(E-4-chloro-but-2-enyl )-3β-(4-methylphenyl)nortropane-2β-carboxylic methyl ester.

8. A method for preparing a [¹⁸F]radiolabelled compound of formula (I): 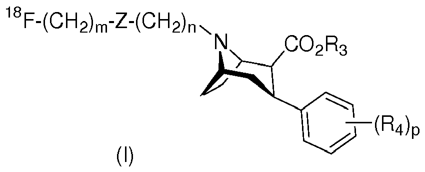 wherein Z, R₃, R₄, m, n, and p are as defined in claims 1 to 7,
comprising the steps of :
i) reacting a compound of formula (III)
X-(CH₂)ₘ-Z-(CH₂)ₙ-Y
wherein Y is Cl, Br, I or OSO₂R₅, X, Z, R₅, m and n being as defined in claim 1 to 7,
with a compound of formula (IV) 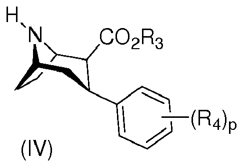 wherein R₃, R₄, and p are as defined in claims 1 to 7,
in the presence of a base in an appropriate solvent; and optionally
ii) recovering the obtained compound of formula (II) as defined in any of claims 1 to 7,
iii) contacting the compound of formula (II),
with a source of ¹⁸F anion in an appropriate solvent ; and optionally
iv) recovering the obtained [¹⁸F]radiolabelled compound of formula (I).

9. The method of claim 8, wherein the source of ¹⁸F anion is an alkaline metal [¹⁸F]fluoride.

10. The method of claim 9, wherein the source of ¹⁸F anion is an alkaline metal [¹⁸F]fluoride -cryptate complex.

11. The method of claim 10, wherein the source of ¹⁸F anion is a potassium [¹⁸ F]fluoride-cryptate complex.

12. The method of claim 11, wherein the source of ¹⁸F anion is a potassium [¹⁸F]fluoride-4,7,13,16,21,24-hexaoxa-1,10-diazabicyclo[8,8,8]hexacosane.

13. A compound of formula (IIIa): 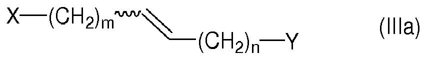 wherein X is Cl, Br, I and Y is OSO₂R₅, m, n and R₅ being as defined in claim 1.

14. The compound of claim 13, wherein Y is OSO₂(C₆H₄)CH₃

15. The compound of any of claims 13 or 14 wherein X is Cl.

16. A use of a compound of formula (II) as defined in claim 1 for preparing a compound of formula (I) as defined in claim 8.

17. A kit for the preparation of a [¹⁸F]radioactively labelled compound of formula (I), comprising:
(a) a compound of formula (II), as defined in claims 1 to 7 ; and
(b) a source of ¹⁸F anion.

## Patentansprüche

1. Eine Verbindung der Formel (II) 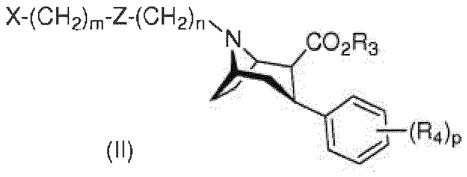 wobei:
- X Cl, Br, I oder eine OSO₂R₅-Gruppe ist,
- Z R₁C=CR₂ oder C=C ist,
- R₁, R₂ jeweils unabhängig H, C₁-C₆ Alkyl, C₆-C₁₀ Aryl sind, wobei die besagten Alkyl- oder Arylgruppen optional mit einer bis drei R₆ Gruppen substituiert sind,
- R₃ H oder C₁-C₆ Alkyl ist, wobei die besagte Alkylgruppe optional mit einer bis drei R₆ Gruppen substituiert ist,
- p 1, 2 oder 3 ist,
- R₄ unabhängig H, Cl, Br, I, F oder C₁-C₆ Alkyl ist/sind, wobei die besagte/n Alkylgruppe/n optional mit einer bis drei R₆ Gruppen substituiert ist/sind,
- R₅ ein C₁-C₆ Alkyl ist, wobei das besagte Alkyl optional mono-, poly- oder perhalogeniert ist, ein C₃-C₈ Cycloalkyl ist oder ein C₆-C₁₂ Aryl ist, wobei das besagte Alkyl, Cycloalkyl und Aryl optional mit einer bis drei C₁-C₄ Alkylgruppen oder F, Cl, Br, NO₂ oder CN substituiert sind,
- R₆ OH, F, Cl, Br, Phenyl oder Methyl ist,
- m 1, 2, 3 oder 4 ist und
- n 1, 2, 3 oder 4 ist,
und deren stereoisomere Formen, Mischungen von stereoisomeren Formen und Salzformen davon.

2. Die Verbindung nach Anspruch 1, wobei X Cl, Br, I ist.

3. Die Verbindung nach Anspruch 2, wobei X Cl ist.

4. Die Verbindung nach einem der Ansprüche 1 bis 3, wobei Z R₁C=CR₂ ist.

5. Die Verbindung nach Anspruch 4, wobei Z trans ist.

6. Die Verbindung nach einem der Ansprüche 1 bis 5, welche von der Formel (IIa) ist: 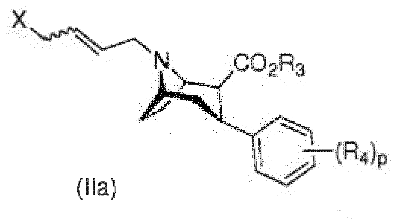 wobei X, R₃, R₄ und p wie in den Ansprüchen 1 bis 3 definiert sind.

7. Die Verbindung nach Anspruch 6, welche der N-(E-4-Chlor-but-2-enyl)-3β-(**4**-methylphenyl)nortropan-2β-Carbonsäuremethylester ist.

8. Ein Verfahren zum Herstellen einer [¹⁸F] radioaktiv markierten Verbindung der Formel (I): 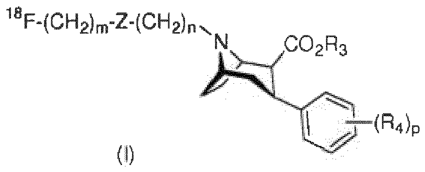 wobei Z, R₃, R₄, m, n und p wie in den Ansprüchen 1 bis 7 definiert sind, welches die Schritte aufweist:
i) Umsetzen einer Verbindung der Formel (III)
X-(CH₂)ₘ-Z-(CH₂)ₙ-Y
wobei Y Cl, Br, I oder OSO₂R₅ ist, und X, Z, R₅, m und n wie in den Ansprüchen 1 bis 7 definiert sind,
mit einer Verbindung der Formel (IV) 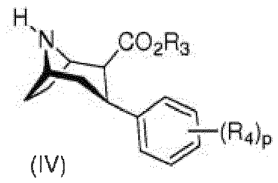 wobei R₃, R₄ und p wie in den Ansprüchen 1 bis 7 definiert sind, in Anwesenheit einer Base in einem geeigneten Lösungsmittel, und optional
ii) Gewinnen der erhaltenen Verbindung der Formel (II), welche wie in einem der Ansprüche 1 bis 7 definiert ist,
iii) Kontaktieren der Verbindung der Formel (II),
mit einer ¹⁸F-Anionenquelle in einem geeigneten Lösungsmittel, und optional
iv) Gewinnen der erhaltenen [¹⁸F] radioaktiv markierten Verbindung der Formel (I).

9. Das Verfahren nach Anspruch 8, wobei die ¹⁸F-Anionenquelle ein Alkalimetall[¹⁸F]fluorid ist.

10. Das Verfahren nach Anspruch 9, wobei die ¹⁸F-Anionenquelle ein Alkalimetall[¹⁸F]fluorid-Kryptatkomplex ist.

11. Das Verfahren nach Anspruch 10, wobei die ¹⁸F-Anionenquelle ein Kalium[¹⁸F]fluorid-Kryptatkomplex ist.

12. Das Verfahren nach Anspruch 11, wobei die ¹⁸F-Anionenquelle ein Kalium[¹⁸F]fluorid-4,7,13,16,22,24-hexaoxa-1,10-diazabicyclo[8,8,8]hexacosan ist.

13. Eine Verbindung der Formel (IIIa): 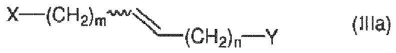 wobei X Cl, Br, I ist und Y OSO₂R₅ ist und m, n und R₅ wie in Anspruch 1 definiert sind.

14. Die Verbindung nach Anspruch 13, wobei Y OSO₂(C₆H₄)CH₃ ist.

15. Die Verbindung nach einem der Ansprüche 13 oder 14, wobei X Cl ist.

16. Eine Verwendung einer Verbindung der Formel (II), welche wie in Anspruch 1 definiert ist, zum Herstellen einer Verbindung der Formel (I), welche wie in Anspruch 8 definiert ist.

17. Ein Kit zur Herstellung einer [¹⁸F] radioaktiv markierten Verbindung der Formel (I), aufweisend:
(a) eine wie in den Ansprüchen 1 bis 7 definierte Verbindung der Formel (II), und
(b) eine ¹⁸F-Anionenquelle.

## Revendications

1. Composé de formule (II) 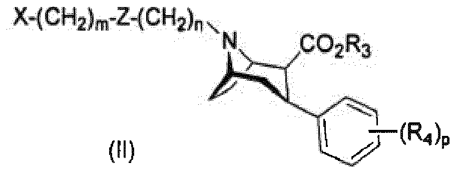 dans laquelle :
- X est CI, Br, I, ou un groupe -OSO₂R₅ ;
- Z est R₁C=CR₂ ou C≡C ;
- chacun de R₁ et R₂ est indépendamment H ou un alkyle en C₁ à C₆ ou aryle en C₆ à C₁₀, lesdits groupes alkyle ou aryle étant éventuellement substitués par un à trois groupes R₆ ;
- R₃ est H ou un alkyle en C₁ à C₆, ledit groupe alkyle étant éventuellement substitué par un à trois groupes R₆ ;
- p vaut 1, 2 ou 3 ;
- le ou les R₄ sont indépendamment H, CI, Br, I, F ou un alkyle en C₁ à C₆, ledit ou lesdits groupes alkyle étant éventuellement substitués par un à trois groupes R₆ ;
- R₅ est un alkyle en C₁ à C₆, ledit alkyle étant éventuellement mono-, poly- ou per-halogéné, un cycloalkyle en C₃ à C₈ ou un aryle en C₆ à C₁₂, lesdits alkyle, cycloalkyle et aryle étant éventuellement substitués par un à trois groupes alkyle en C₁ à C₄ ou F, Cl, Br, NO₂ ou CN ;
- R₆ est OH, F, Cl, Br, phényle ou méthyle,
- m vaut 1, 2, 3 ou 4, et
- n vaut 1, 2, 3 ou 4,
et ses formes stéréoisomères, des mélanges de formes stéréoisomères, et les formes sels de ceux-ci.

2. Composé selon la revendication 1, dans lequel X est Cl, Br, I.

3. Composé selon la revendication 2, dans lequel X est Cl.

4. Composé selon l'une quelconque des revendications 1 à 3, dans lequel Z est R₁C=CR₂.

5. Composé selon la revendication 4, dans lequel Z est *trans.*

6. Composé selon l'une quelconque des revendications 1 à 5, qui est de formule (IIa) : 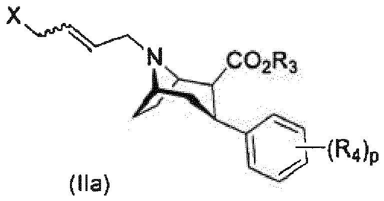 dans laquelle X, R₃, R₄ et p sont tels que définis dans les revendications 1 à 3.

7. Composé selon la revendication 6, qui est l'ester méthylique d'acide N-(E-4-chlorobut-2-ényl)-3β-(**4**-méthylphényl)nortropane-2β-carboxylique.

8. Procédé pour préparer un composé [¹⁸F]-radiomarqué de formule (I) : 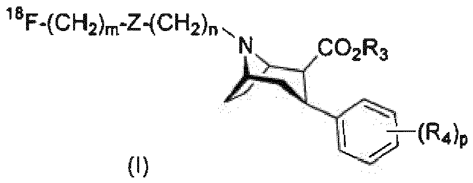 dans laquelle Z, R₃, R₄, m, n et p sont tels que définis dans les revendications 1 à 7, comprenant les étapes de :
i) réaction d'un composé de formule (III)
X-(CH₂)ₘ-Z-(CH₂)ₙ-Y
dans laquelle Y est Cl, Br, I ou OSO₂R₅, et X, Z, R₅, m et n sont tels que définis dans les revendications 1 à 7,
avec un composé de formule (IV) 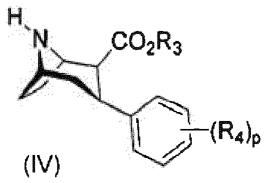 dans laquelle R₃, R₄ et p sont tels que définis dans les revendications 1 à 7,
en présence d'une base dans un solvant approprié ; et éventuellement
ii) récupération du composé obtenu de formule (II) tel que défini dans l'une quelconque des revendications 1 à 7,
iii) mise en contact du composé de formule (II) avec une source d'anion ¹⁸F dans un solvant approprié ; et éventuellement
iv) récupération du composé [¹⁸F]-radiomarqué de formule (I).

9. Procédé selon la revendication 8, dans lequel la source d'anion ¹⁸F est un [¹⁸F]-fluorure de métal alcalin.

10. Procédé selon la revendication 9, dans lequel la source d'anion ¹⁸F est un complexe de cryptate et de [¹⁸F]-fluorure de métal alcalin.

11. Procédé selon la revendication 10, dans lequel la source d'anion ¹⁸F est un complexe de cryptate et de [¹⁸F]-fluorure de potassium.

12. Procédé selon la revendication 11, dans lequel la source d'anion ¹⁸F est un complexe de 4,7,13,16,21,24-hexaoxa-1,10-diazabicyclo[8,8,8]hexacosane et de [¹⁸F]-fluorure de potassium.

13. Composé de formule (IIIa) : 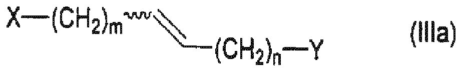 dans laquelle X est CI, Br, I, Y est SO₂R₅, et m, n et R₅ sont tels que définis dans la revendication 1.

14. Composé selon la revendication 13, dans lequel Y est OSO₂(C₆H₄)CH₃.

15. Composé selon l'une quelconque des revendications 13 et 14, dans lequel X est CI.

16. Utilisation d'un composé de formule (II) tel que défini dans la revendication 1 pour préparer un composé de formule (I) tel que défini dans la revendication 8.

17. Kit pour la préparation d'un composé [¹⁸F]-radiomarqué de formule (I), comprenant :
(a) un composé de formule (II) tel que défini dans les revendications 1 à 7 ; et
(b) une source d'anion ¹⁸F.
